(19) Europäisches Patentamt
European Patent Office
Office européen des brevets

(11) **EP 1 072 250 B1**

(12) **EUROPÄISCHE PATENTSCHRIFT**

(45) Veröffentlichungstag und Bekanntmachung des Hinweises auf die Patenterteilung:
**15.09.2004 Patentblatt 2004/38**

(51) Int Cl.[7]: **A61K 7/06**, A61K 7/00, B65D 83/48

(21) Anmeldenummer: **00113653.0**

(22) Anmeldetag: **28.06.2000**

(54) **Wasserhaltiges Aerosol-Haarspray mit einem reduzierten Gehalt an leichtflüchtigen organischen Bestandteilen**

Aqueous aerosol-hairspray having a reduced content of volatile organic matter

Aérosol capillaire aqueux ayant un contenu réduit de matières volatiles organiques

(84) Benannte Vertragsstaaten:
**AT BE CH CY DE DK ES FI FR GB GR IE IT LI LU MC NL PT SE**

(30) Priorität: **23.07.1999 DE 19934701**

(43) Veröffentlichungstag der Anmeldung:
**31.01.2001 Patentblatt 2001/05**

(73) Patentinhaber: **Wella Aktiengesellschaft 64274 Darmstadt (DE)**

(72) Erfinder:
• **Stähle, Liane 64372 Ober-Ramstadt (DE)**
• **Herfurt, Ulrich 55127 Mainz (DE)**

(56) Entgegenhaltungen:
EP-A- 0 791 351    DE-A- 3 434 886
FR-A- 1 479 267    FR-A- 2 749 568
US-A- 3 790 089    US-A- 4 230 243
US-A- 4 243 548

**Beschreibung**

Gegenstand der Erfindung ist ein wasserhaltiges Aerosol-Haarspray mit einem Gehalt an haarfestigenden Polymeren und einer speziell ausgewählten Mischung von Lösungsmitteln und Treibmitteln, wobei der Anteil an als VOC (volatile organic compounds, leichtflüchtige organische Bestandteile) eingestuften Stoffen vorzugsweise maximal 55% beträgt.

Aerosol-Haarsprays wurden und werden üblicherweise in Form von Polymerlösungen oder Polymerdispersionen in alkoholischem Milieu verwendet. Diese herkömmlichen, hohen Anteile an VOC enthaltenden Haarsprays zeichnen sich durch hervorragende Produkt- und Anwendungseigenschaften aus. Aufgrund von Umweltschutzgesichtspunkten sowie zu erwartender gesetzlicher Reglementierungen besteht ein Bedarf, in Haarsprays den Gehalt an VOC, zu denen u.a. auch die bisher in Haarsprays üblichen alkoholischen Lösungsmittel wie Ethanol und Isopropanol sowie übliche Treibmittel wie z.B. Propan, Butan oder Dimethylether gehören, möglichst weit zu reduzieren.

Es sind bereits Produkte mit einem VOC-Anteil von maximal 80% bekannt. Bei diesen 80% VOC-Formulierungen sind die Gebrauchseigenschaften jedoch in der Regel deutlich schlechter als diejenigen von herkömmlichen Haarsprays und die Performance ist nur noch gerade akzeptabel bis mangelhaft (Aerosol and Spray Report, Vol. 35, No.5/96, Seite 254 ff.). Es werden weniger Halt, mehr Beanstandungen und ein genereller Marktrückgang beobachtet. Für 55% VOC-Formulierungen wird eine Zunahme der Probleme erwartet und es wird erwartet, daß entweder eine weitere Verschlechterung der Gebrauchseigenschaften oder eine deutliche Erhöhung der Kosten in Kauf genommen werden muß. Bekannte Lösungsansätze für 55% VOC-Formulierungen gehen entweder von einem erhöhten Wassergehalt in Kombination mit Dimethylether oder Propan/Butan oder einem Dimethylether/Butan-Gemisch als Treibmittel oder von wasserfreien Formulierungen in Kombination mit den als non-VOC eingestuften Treibmitteln 1,1-Difluorethan oder 1,1,1,2-Tetrafluorethan aus. Die bisher bekannten 55% VOC-Formulierungen reichen aber noch nicht an die hervorragenden Gebrauchseigenschaften herkömmlicher, wasserfreier Haarsprays mit hohem VOC-Gehalt heran. Ein erhöhter Wassergehalt führt zu vielfältigen Problemen, z.B. schlechterer Halt der Frisur, erhöhter Curl Droop Effekt, hoher Nässegrad, erhöhte Klebrigkeit des Polymerfilms während der Trockenphase, erhöhte Tröpfchengröße des Sprays, verschlechtertes Sprühbild, Schaumbildung beim Versprühen, erhöhte Viskosität und damit verbundene schlechtere Versprühbarkeit, erhöhte Trockenzeit, Instabilitäten gegenüber Hydrolyse, Korrosion, schlechtere Verträglichkeit mit Solventien, Co-Solventien, Treibmitteln und/oder Polymeren, was sich in einer schlechteren Lagerstabilität, einer schlechteren Kältestabilität oder der Bildung von Trübungen, Ausfällungen oder mehreren flüssigen Phasen bemerkbar macht. Die Hauptkriterien Haarfestigung, Haarvernetzung, Griff (Rauhigkeit, Klebrigkeit, Glattheit) und Trockenzeit der wäßrigen oder wäßrig-alkoholischen Haarsprays wurden sowohl von Endverbrauchern als auch von Friseuren bisher immer als unakzeptabel schlechter beurteilt. Die ausschließliche Verwendung von fluorierten Kohlenwasserstoffen als non-VOC Treibmittel in wasserfreien Formulierungen hat den Nachteil, daß die Sprays wegen des hohen Preises der fluorierten Kohlenwasserstoffe sehr kostenintensiv sind.

Aceton als Lösungsmittelersatz für die sonst üblichen niederen Alkohole ist kein gängiger Bestandteil von Haarsprayformulierungen (Aerosol and Spray Report, Vol. 37, No. 6/98, Seite 18), da Aceton eine ganze Reihe von gravierenden Nachteilen hat: es riecht unakzeptabel, was auch durch Parfümierung nicht überdeckt werden kann; es verdampft zu schnell, was zur Folge hat, daß die aufgesprühte Lösung nicht ausreichend auf dem Haar verläuft und somit eine schlechtere Vernetzung und eine schlechtere Festigung resultiert; gut in Wasser oder in Wasser/Alkohol lösliche Polymere sind in Wasser/Aceton-Mischungen nicht oder nur unzureichend löslich; in Wasser/Aceton lösliche Polymere sind in Wasser häufig schlecht löslich und fallen daher bei der schnellen Verdampfung von Aceton auf dem Haar zu schnell aus, bevor sich ein Film bilden kann, was eine schlechtere Festigung zur Folge hat. In der EP 791 351 A1 werden kosmetische Zusammensetzungen beschrieben, welche ein festigendes Polymer, Aceton, einen C1- bis C4-Alkohol, einen linearen C5- bis C8-Kohlenwasserstoff und Wasser enthalten. Die Zusammensetzungen können miteinem Treibmittel wie z.B. Dimethylether in Form eines Aerosolhaarsprays vorliegen.

Es bestand daher die Aufgabe, die Eigenschaften von wasserhaltigen Aerosol-Haarsprays mit einem reduzierten VOC-Gehalt weiter zu optimieren und möglichst an die Eigenschaften der herkömmlichen, wasserfreien Haarsprays mit hohem VOC-Gehalt heranzuführen ohne die Formulierungskosten allzusehr zu erhöhen.

Es wurde nun gefunden, daß die Aufgabe gelöst wird durch ein wasserhaltiges Aerosol-Haarspray mit einem Gehalt an haarfestigenden Polymeren und einer speziellen Kombination von bestimmten, ausgewählten Lösungsmitteln und Treibmitteln, vorzugsweise in bestimmten, ausgewählten Mengen.

Gegenstand der Erfindung ist daher ein Aerosol-Haarspray bestehend aus einer Haarsprayzusammensetzung, einem Druckbehälter und einer Vorrichtung zum Versprühen, wobei die Zusammensetzung einen Gehalt aufweist an

(A) mindestens einem haarfestigenden Polymer,
(B) 10 bis 40 Gew.% mindestens eines Alkohols mit 1 bis 4 Kohlenstoffatomen,
(C) 10 bis 30 Gew.% Wasser,

(D) 1 bis 30 Gew.% mindestens eines Co-Lösungsmittels, ausgewählt aus Aceton, Methylacetat oder deren Gemisch,

(E) 20 bis 75 Gew.% einer Treibmittelmischung aus Dimethylether und fluorierten Kohlenwasserstoffen.

Der Gegenstand der vorliegenden Erfindung ist hervorragend dazu geeignet, um Formulierungen mit einem reduzierten Anteil an leicht flüchtigen organischen Bestandteilen (VOC), beispielsweise VOC 80% oder VOC 55% Formulierungen herzustellen. Das erfindungsgemäße Mittel enthält daher vorzugsweise maximal 80 Gewichtsprozent, besonders bevorzugt maximal 55 Gewichtsprozent an Bestandteilen, welche als VOC klassifiziert sind. Das California Air Resources Board (CARB) definiert die VOC als Stoffe mit einem Dampfdruck von > 0,1 mm Hg bei 20°C oder als Stoffe mit 12 oder weniger Kohlenstoffatomen, wobei eine Reihe von Substanzen bei dieser Definition wegen ihres geringen oder nicht vorhandenen photochemischen Ozonbildungspotentials (Photochemical Ozone Creation Potential, POCP) ausgenommen sind, z.B. Kohlendioxid, Methylenchlorid, Aceton, Methylacetat, FCKWs und fluorierte Kohlenwasserstoffe.

Das haarfestigende Polymer ist vorzugsweise in einer Menge von 3 bis 20 Gew.%, besonders bevorzugt von 5 bis 10 Gew.% enthalten. Polymere, die verwendet werden können, sind in dem erfindungsmäßen Lösungsmittel-/Treibmittelgemisch lösliche oder dispergierbare nichtionische, anionische oder amphotere Polymere, insbesondere Copolymere, welche aus mindestens einer hydrophilen und mindestens einer hydrophoben Monomerart aufgebaut sind.

Geeignete anionische Polymere sind synthetische Homooder Copolymere mit neutralisierbaren Säuregruppen enthaltenden Monomereinheiten, welche gegebenenfalls mit Comonomeren, die keine Säuregruppen enthalten, copolymerisiert sind. Als Säuregruppen kommen Sulfonsäure-, Phosphorsäure- und Carbonsäuregruppen in Betracht, von denen die Carbonsäuregruppen bevorzugt sind. Geeignete Säuregruppen enthaltende Monomere sind beispielsweise Acrylsäure, Methacrylsäure, Crotonsäure, Maleinsäure bzw. Maleinsäureanhydrid, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes anionisches, natürliches Polymer ist beispielsweise teilweise oder vollständig neutralisierter Schellack.

Bevorzugte Polymere mit Säuregruppen sind vernetzte oder unvernetzte Vinylacetat/Crotonsäure Copolymere, die beispielsweise in Form einer 60%igen Lösung in Isopropanol/Wasser unter der Handelsbezeichnung ARISTOFLEX® von der Firma HOECHST/Deutschland beziehungsweise von der Firma BASF unter dem Handelsnamen LWISET® CA-66 vertrieben werden. Weitere geeignete anionische Polymere sind zum Beispiel Terpolymere aus Vinylacetat, Crotonsäure und Polyethylenoxid sowie Terpolymere aus Acrylsäure, Alkylacrylat und N-Alkylacrylamid, insbesondere Acrylsäure/ Ethylacrylat/N-t-Butylacrylamid Terpolymere, wie sie unter den Handelsnamen ULTRAHOLD® 8 und ULTRAHOLD® STRONG der Firma BASF/Deutschland vertrieben werden oder Terpolymere aus Vinylacetat, Crotonat und Vinylalkanoat, insbesondere Vinylacetat/Crotonat/Vinylneodecanoat Copolymere, wie sie z. B. von der Firma National Starch unter der Handelsbezeichnung RESYN 28-2930 vertrieben werden oder Vinylacetat/Crotonat/Vinylpropionat Copolymere.

Eine weitere Klasse von geeigneten anionischen Polymeren sind anionische Polyurethane. Bevorzugte Polyurethane sind dadurch gekennzeichnet, daß sie (a) endständige Säuregruppen besitzen, die beispielweise über Aminosulfonsäuren oder Aminocarbonsäuren eingeführt wurden, (b) gegebenenfalls weitere freie Carbonsäuregruppen enthalten, die durch Einpolymerisieren von Carbonsäurediolen wie beispielsweise Dimethylolpropansäure als Comonomere eingeführt wurden und (c) Polyurethansequenzen enthalten, die aus Polyesterdiolen und Diisocyanaten wie beispielsweise Alkylendiisocyanaten oder Isophorondiisocyanat gebildet wurden. Geeignet ist beispielsweise Luviset® PUR der Firma BASF/Deutschland.

Die anionischen Polymere liegen im erfindungsgemässen Mittel teilweise oder vollständig mit einem kosmetisch verträglichen Neutralisationsmittel neutralisiert vor. Als Neutralisationsmittel können organische oder anorganische Basen verwendet werden. Beispiele für Basen sind insbesondere Aminoalkanole wie z.B. Aminomethylpropanol (AMP), Triethanolamin oder Monoethanolamin, aber auch Ammoniak, NaOH u.a..

Geeignete nichtionische, haarfestigende Polymere sind zum Beispiel Homo- oder Copolymere, welche aus mindestens einem nichtionischen Monomer aufgebaut sind. Nichtionische Monomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignete synthetische, nichtionische, haarfestigende Polymere sind zum Beispiel Homopolymere des Vinylpyrrolidons sowie Homopolymere des N-Vinylformamids. Weitere geeignete synthetische filmbildende, nichtionische haarfestigende Polymere sind zum Beispiel Copolymerisate aus Vinylpyrrolidon und Vinylacetat, Terpolymere aus Vinylpyrrolidon, Vinylacetat und Vinylpropionat, Polyacrylamide, Polyvinylalkohole oder Polyethylenglykole mit einem Molekulargewicht von 800 bis 20.000 g/mol.

Geeignete amphotere Polymere sind Polymere, welche sowohl kationische oder durch Protonierung kationisierbare Gruppen als auch anionische oder durch Deprotonierung anionisierbare Gruppen enthalten. Kationische Gruppen sind beispielsweise quaternäre Amingruppen, kationisierbare Gruppen sind beispielsweise primäre, sekundäre oder tertiäre Amingruppen. Anionische Gruppen sind beispielsweise Carboxylat-, Sulfat-, Sulfonat-, Phosphat- oder Phosphonatgruppen. Anionisierbare Gruppen sind beispielsweise die protonierten Formen der genannten anionischen Gruppen.

Geeignete amphotere Polymere sind z.B. Copolymere aus Octylacrylamid, t-Butylaminoethylmethacrylat und zwei oder mehr Monomeren, bestehend aus Acrylsäure, Methacrylsäure oder deren Estern, wobei mindestens eines der Acrylatmonomere eine nicht-veresterte Säuregruppe trägt.

Als in dem erfindungsgemäßen Haarspray am besten einsetzbar haben sich die folgenden haarfestigenden Polymere erwiesen: Polyvinylpyrrolidon/Vinylacetat Copolymer, Copolymere aus Octylacrylamid, t-Butylaminoethylmethacrylat und zwei oder mehr Monomeren, bestehend aus Acrylsäure, Methacrylsäure oder deren Estern, wobei mindestens eines der Acrylatmonomere eine nicht-veresterte Säuregruppe trägt (Amphomer®), Vinylpyrrolidon/Vinylcaprolactam/Dialkylaminoalkylmethacrylamid Copolymere und Acrylat Copolymere, bei denen eine erste hydrophile Monomerart ausgewählt ist aus Acrylsäure und Methacrylsäure oder hydroxysubstituierten Estern der Acryl- oder Methacrylsäure und eine zweite, hydrophobe Monomerart ausgewählt ist aus Estern der Acrylsäure oder der Methacrylsäure (INCI: Acrylates Copolymer, Acrylates/Hydroxyester Acrylate Copolymer), beispielsweise ein Terpolymer aus Butylacrylat, Methylmethacrylat und Methacrylsäure oder ein Terpolymer aus Butylacrylat, Methylmethacrylat und 2-Hydroxyethylmethacrylat.

Besonders bevorzugte Polymere sind die Polymere mit der INCI-Bezeichnung Acrylates Copolymers und einer Acidität von 1,4 bis 1,6 meq/g, beispielsweise Balance® CR oder Balance® Extra von National Starch, welches in Form einer 45%igen wäßrigen Emulsion vertrieben wird oder die Polymere mit der INCI-Bezeichnung Acrylates/Hydroxyester Acrylate Copolymer, beispielsweise Acudyne® 258 von Rohm & Haas. Weitere, besonders bevorzugte haarfestigende Polymere sind Terpolymere aus Vinylpyrrolidon, Vinylcaprolactam und Dimethylaminopropylmethacrylamid (DMAPMA) mit einem Gehalt an 1-25 Gew.%, vorzugsweise 5 bis 15 Gew.% Vinylpyrrolidon, 60 bis 95 Gew.%, vorzugsweise 80 bis 90 Gew.% Vinylcaprolactam und 1 bis 10 Gew.%, vorzugsweise 2,5 bis 7,5 Gew.% DMAPMA. Die Synthese dieser Terpolymere wird in WO96/19971 beschrieben. Ein geeignetes Terpolymer ist beispielsweise Aquaflex SF-40® von ISP, welches mit einem Feststoffgehalt von 40% in Ethanol vertrieben wird.

Erfindungsgemäß wird ein Lösungsmittelgemisch aus Wasser, niederen Alkoholen und bestimmten Co-Lösungsmitteln eingesetzt, dessen Eigenschaften durch die teilweise Löslichkeit der Treibmittel Dimethylether (bis zu 35% in Wasser) und der fluorierten Kohlenwasserstoffe (1,1-Difluorethan bis zu 1,7% in Wasser) weiter modifiziert wird. Der C1- bis C4-Alkohol ist in einer Menge von 10 bis 40 Gew.%, bevorzugt von 20 bis 30 Gew.%, besonders bevorzugt von 25 bis 30 Gew.% enthalten. Alkohole sind beispielsweise Methanol, Ethanol, Isopropanol, n-Propanol und Butanol, von denen Ethanol und Isopropanol besonders bevorzugt sind.

Der Wassergehalt beträgt 10 bis 30 Gew.%, vorzugsweise 15 bis 25 Gew.%, besonders bevorzugt 20 bis 25 Gew.%.

Als Co-Lösungsmittel wird Aceton, Methylacetat oder deren Gemisch in einer Menge von 1 bis 30 Gew.%, vorzugsweise von 5 bis 15 Gew.%, besonders bevorzugt von 5 bis 10 Gew.% eingesetzt. Besonders bevorzugt ist Aceton. Aus den unten beschriebenen Vergleichsversuchen ergeben sich eine ganze Reihe von positiven Effekten durch die Verwendung von Aceton in wäßrig-alkoholischen Aerosol-Haarsprays. Es war überraschend, daß nicht nur die oben erwähnten negativen Eigenschaften des Acetons in dem erfindungsgemäßen Lösungsmittel/Treibmittelsystem unterdrückt werden, sondern zusätzlich eine Reihe von Verbesserungen erzielt werden, insbesondere hinsichtlich Tropfenbildung, Tropfengröße, Feuchtigkeit, Trockenzeit und Anfühlen des Polymerfilms.

Als Treibmittel sind primär die üblichen, bekannten Aerosol-Treibmittel geeignet, beispielsweise niedere Alkane, wie zum Beispiel n-Butan, i-Butan und Propan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel.

Als Treibmittelsystem wird eine Mischung aus Dimethylether und fluorierten Kohlenwasserstoffen eingesetzt, dessen Eigenschaften insbesondere in Hinblick auf Tröpfchengröße und Trockenzeit durch die bei Raumtemperatur nicht unerheblichen Dampfdrücke der als Lösungsmittel eingesetzten niederen Alkohole und des Co-Lösungsmittels weiter modifiziert werden. Der Gehalt an Dimethylether beträgt 15 bis 45 Gew.%, vorzugsweise von 25 bis 35 Gew. %, besonders bevorzugt von 30 bis 35 Gew.%. Der Gehalt an fluorierten Kohlenwasserstoffen beträgt 5 bis 30 Gew. %, vorzugsweise 5 bis 15 Gew.%, besonders bevorzugt 5 bis 10 Gew.%. Geeignete fluorierte Kohlenwasserstoffe sind 1,1-Difluorethan und 1,1,1,2-Tetrafluorethan, von denen das 1,1-Difluorethan bevorzugt ist. Das Verhältnis von Dimethylether zu fluoriertem Kohlenwasserstoff liegt dabei idealerweise im Bereich von 1,5:1 bis 9:1, bevorzugt von 3:1 bis 7:1, besonders bevorzugt von 3:1 bis 5:1 bei einem Gesamttreibmittelgehalt von 20 bis 75 Gew.%, bevorzugt von 30 bis 55 Gew.%, besonders bevorzugt von 35 bis 45 Gew.%.

Die erfindungsgemäße Haarsprayzusammensetzung wird in einen handelsüblichen Druckbehälter abgefüllt, welcher entweder aus korrosionsfreiem Material besteht oder zur Vermeidung von Korrosion innen beschichtet ist oder mindestens einen Korrosionsinhibitor enthält.

Die besten Ergebnisse werden mit dem erfindungsgemäßen Haarspray erzielt, wenn das Sprühsystem so ausgelegt wird, daß die Produktaustrittsrate von 3,5 bis 5,5 g, vorzugsweise von 4 bis 5 g pro 10 Sekunden Sprühdauer beträgt und ein Ventil mit Seitenbohrung (Vapor Phase-Gehäuse) sowie ein Sprühkopf mit Soft-Wirbeldüse verwendet wird.

Die erfindungsgemäße Haarsprayzusammensetzung kann weitere, übliche kosmetische Zusatzstoffe enthalten, zum Beispiel Weichmacher wie Glycerin, Glykol oder Phtalatester; Duftstoffe und Parfümöle, Lichtschutzmittel, UV-Filter, haarpflegende Zusätze, Kämmbarkeitsverbesserer, Feuchthaltemittel, Farbstoffe, Korrosionsinhibitoren, Antioxidantien und Konservierungsstoffe in einer Menge von jeweils 0,01 bis 10 Gew.% und einer Gesamtmenge von 0,01 bis 20 Gew.%.

Das erfindungsgemäße Haarspray zeichnet sich dadurch aus, daß es die Herstellung von kostengünstigen Aerosol-Haarsprays mit einem Gehalt an maximal 55% VOCs bei gleichzeitig guten Anwendungseigenschaften ermöglicht. Es weist einen relativ geringen Gehalt der vergleichsweise teuren non-VOC Treibmittel fluorierte Kohlenwasserstoffe auf bei dennoch guten Anwendungseigenschaften, insbesondere einen nicht zu hohen Nässegrad, einen verringerten Curl Droop Effekt und akzeptable Trocknungsgeschwindigkeiten.

Die nachfolgenden Beispiele sollen den Gegenstand der Erfindung näher erläutern:

**Beispiele**

Die in den folgenden Beispielen angegebenen Polymermengen beziehen sich jeweils auf den Feststoffgehalt.

**Beispiel 1: VOC 55% Haarspray Aerosol**

| | |
|---|---|
| 6,0 g | PVP/Vinylcaprolactam/DMAPA/Acrylat Copolymer (Aquaflex® SF 40 von ISP) |
| 5,0 g | Aceton |
| 25,0 g | Ethanol |
| 24,0 g | Wasser |
| 30,0 g | Dimethylether |
| 10,0 g | HFKW 152a (1,1-Difluorethan) |
| 100,0 g | |

Die Zusammensetzung wird in eine Aerosoldose abgefüllt, mit einem Ventil mit Vapor Phase Gehäuse (Fa. Precision, Gehäuse .050" x .013") und mit einem Kapillarsteigrohr (.060"/.133") versehen. Die Kegelbohrung beträgt 1x.013". Als Sprühkopf wird ein Sprühkopf mit Soft-Wirbeldüse .016" eingesetzt. Die Produktaustrittsrate beträgt 4,5 g bei 10 s Sprühdauer.

**Beispiel 2: VOC 55% Haarspray Aerosol**

| | |
|---|---|
| 6,0 g | PVP/VA |
| 10,0 g | Aceton |
| 25,0 g | Ethanol |

(fortgesetzt)

| 14,0 g | Wasser |
|---|---|
| 30,0 g | Dimethylether |
| 15,0 g | HFKW 152a (1,1-Difluorethan) |
| 100,0 g | |

Die Abfüllung erfolgte wie bei Beispiel 1.

**Beispiel 3: VOC 55% Haarspray Aerosol**

| 5,0 g | Acrylates Copolymer (Balance® Extra von National Starch) |
|---|---|
| 0,3 g | Aminomethylpropanol |
| 15,0 g | Aceton |
| 30,0 g | Ethanol |
| 19,7 g | Wasser |
| 25,0 g | Dimethylether |
| 5,0 g | HFKW 134a (Tetrafluorethan) |
| 100,0 g | |

Die Abfüllung erfolgte wie bei Beispiel 1.

**Beispiel 4: VOC 55% Haarspray Aerosol**

| 7,0 g | Acrylates/Hydroxyester Acrylate Copolymer (Acudyne® 258 von Rohm & Haas) |
|---|---|
| 0,7 g | Aminomethylpropanol |
| 5,0 g | Methylacetat |
| 20,0 g | Ethanol |
| 22,3 g | Wasser |
| 35,0 g | Dimethylether |
| 10,0 g | HFKW 152a (1,1-Difluorethan) |
| 100,0 g | |

Die Abfüllung erfolgte wie bei Beispiel 1.

**Beispiel 5: Vergleichsversuch**

Es wurde ein erfindungsgemäßes, Aceton enthaltendes Aerosol-Haarspray 5A verglichen mit einem analogen, nicht erfindungsgemäßen Haarspray 5B ohne Acetonzusatz. Die genauen Zusammensetzungen sind in Tabelle 1 wiedergegeben. Die Abfüllung erfolgte wie bei Beispiel 1.

Tabelle 1

| Untersuchte Zusammensetzungen | | |
|---|---|---|
| | 5A | 5B |
| PVP/Vinylcaprolactam/DMAPA/Acrylat Copolymer (Aquaflex® SF 40 von ISP) | 5 g | 5 g |
| Ethanol | 25 g | 25 g |
| Wasser | 25 g | 30 g |
| Aceton | 5 g | - |

Tabelle 1   (fortgesetzt)

| Untersuchte Zusammensetzungen | | |
|---|---|---|
| | 5A | 5B |
| Dimethylether | 30 g | 30 g |
| HFKW 152a (1,1-Difluorethan) | 10 g | 10 g |

In Halbseitenversuchen wurde jeweils eine Hälfte der Haare einer Testperson mit dem erfindungsgemäßen Haarspray 5A und die andere Hälfte der Haare mit dem nicht erfindungsmäßen Haarspray 5B besprüht. Die besprühten Haare wurden durch ausgebildete Friseurfachkräfte beurteilt. Dabei wurde für alle der nachfolgend genannten Kriterien eine bessere Beurteilung für das erfindungsmäße Spray 5A festgestellt:

Tropfenbildung (positiv: wenig Tropfen auf dem besprühten Haar sichtbar;
    negativ: viele Tropfen sichtbar)
Tropfengröße (positiv: kleine Tropfen;
    negativ: große Tropfen)
Feuchtigkeit (positiv: geringe Anfeuchtung des Haares;
    negativ: große Anfeuchtung des Haares)
Trockenzeit (positiv: kurze Trockenzeit;
    negativ: lange Trockenzeit)
Anfühlen des Harzfilms (Griff)
(positiv: angenehmer, natürlicher Griff;
    negativ: rauher oder klebriger, unnatürlicher Griff)

**Patentansprüche**

1.   Aerosol-Haarspray bestehend aus einer Haarsprayzusammensetzung, einem Druckbehälter und einer Vorrichtung zum Versprühen, wobei die Zusammensetzung einen Gehalt aufweist an

(A) mindestens einem haarfestigenden Polymer,
(B) 10 bis 40 Gew.% mindestens eines Alkohols mit 1 bis 4 Kohlenstoffatomen,
(C) 10 bis 30 Gew.% Wasser,
(D) 1 bis 30 Gew.% mindestens eines flüchtigen Co-Lösungsmittels, ausgewählt aus Aceton, Methylacetat oder deren Gemisch,
(E) 20 bis 75 Gew.% einer Aerosol-Treibmittelmischung aus Dimethylether und fluorierten Kohlenwasserstoffen.

2.   Aerosol-Haarspray nach Anspruch 1, **dadurch gekennzeichnet, daß** als Aerosoltreibmittel 15 bis 45 Gew.% Dimethylether und 5 bis 30 Gew.% mindestens eines fluorierten Kohlenwasserstoffs enthalten sind.

3.   Aerosol-Haarspray nach Anspruch 2, wobei die Zusammensetzung einen Gehalt aufweist an

(A) mindestens einem haarfestigenden Polymer,
(B) 20 bis 30 Gew.% mindestens eines Alkohols mit 1 bis 4 Kohlenstoffatomen,
(C) 15 bis 25 Gew.% Wasser,
(D) 5 bis 15 Gew.% mindestens eines flüchtigen Co-Lösungsmittels, ausgewählt aus Aceton, Methylacetat oder deren Gemisch,
(E) 25 bis 35 Gew.% Dimethylether und 5 bis 15 Gew.% mindestens eines fluorierten Kohlenwasserstoffs.

4.   Haarspray nach einem der vorgenannten Ansprüche, **dadurch gekennzeichnet, daß** das Sprühsystem ein Ventil mit Seitenbohrung aufweist.

5.   Haarspray nach einem der vorgenannten Ansprüche, **dadurch gekennzeichnet, daß** das Sprühsystem so ausgelegt ist, daß die Produktaustrittsrate von 3,5 bis 5,5 g pro 10 Sekunden Sprühdauer beträgt.

**6.** Haarspray nach einem der vorgenannten Ansprüche, **dadurch gekennzeichnet, daß** das Polymer (A) zu 3 bis 20 Gewichtsprozent enthalten ist.

**7.** Haarspray nach einem der vorgenannten Ansprüche, **dadurch gekennzeichnet, daß** der Alkohol (B) ausgewählt ist aus Ethanol und Isopropanol.

**8.** Haarspray nach einem der vorgenannten Ansprüche, **dadurch gekennzeichnet, daß** der fluorierte Kohlenwasserstoff ausgewählt ist aus 1,1-Difluorethan und 1,1,1,2-Tetrafluorethan.

**9.** Haarspray nach einem der vorgenannten Ansprüche, **dadurch gekennzeichnet, daß** als Co-Lösungsmittel (D) Aceton enthalten ist.

**10.** Haarspray nach einem der vorgenannten Ansprüche, **dadurch gekennzeichnet, daß** es nicht mehr als 55 Gewichtsprozent von als VOC eingestuften Stoffen enthält.

**11.** Verwendung von Aceton, Methylacetat oder deren Gemisch zur Verbesserung der Anwendungseigenschaften von wäßrig-alkoholischen Aerosolhaarsprays, welche eine Aerosol-Treibmittelmischung aus Dimethylether und fluorierten Kohlenwasserstoffen enthalten.

**Claims**

**1.** Aerosol hair spray consisting of a hairspray composition, a pressurized container and a spraying device, where the composition has a content of

(A) at least one hair-setting polymer,
(B) 10 to 40% by weight of at least one alcohol with 1 to 4 carbon atoms,
(C) 10 to 30% by weight of water,
(D) 1 to 30% by weight of at least one volatile cosolvent chosen from acetone, methyl acetate or a mixture thereof,
(E) 20 to 75% by weight of an aerosol propellant mixture of dimethyl ether and fluorinated hydrocarbons.

**2.** Aerosol hairspray according to Claim 1, **characterized in that** 15 to 45% by weight of dimethyl ether and 5 to 30% by weight of at least one fluorinated hydrocarbon are present as aerosol propellant.

**3.** Aerosol hairspray according to Claim 2, where the composition has a content of

(A) at least one hair-setting polymer,
(B) 20 to 30% by weight of at least one alcohol having 1 to 4 carbon atoms,
(C) 15 to 25% by weight of water,
(D) 5 to 15% by weight of at least one volatile cosolvent chosen from acetone, methyl acetate or a mixture thereof,
(E) 25 to 35% by weight of dimethyl ether and 5 to 15% by weight of at least one fluorinated hydrocarbon.

**4.** Hairspray according to one of the preceding claims, **characterized in that** the spraying system has a valve with side bore.

**5.** Hairspray according to one of the preceding claims, **characterized in that** the spraying system is designed such that the product exit rate is from 3.5 to 5.5 g per 10 seconds spraying time.

**6.** Hairspray according to one of the preceding claims, **characterized in that** the polymer (A) is present in an amount from 3 to 20% by weight.

**7.** Hairspray according to one of the preceding claims, **characterized in that** the alcohol (B) is chosen from ethanol and isopropanol.

**8.** Hairspray according to one of the preceding claims, **characterized in that** the fluorinated hydrocarbon is chosen from 1,1-difluoroethane and 1,1,1,2-tetrafluoroethane.

9. Hairspray according to one of the preceding claims, **characterized in that** acetone is present as cosolvent (D).

10. Hairspray according to one of the preceding claims, **characterized in that** it comprises not more than 55% by weight of substances classified as VOC.

11. Use of acetone, methyl acetate or a mixture thereof for improving the application properties of aqueous-alcoholic aerosol hairsprays which comprise an aerosol propellant mixture of dimethyl ether and fluorinated hydrocarbons.


**Revendications**

1. Laque pour cheveux en aérosol, constituée d'une composition de laque pour cheveux, d'un contenant sous pression et d'un dispositif pour la pulvérisation, dans laquelle la composition contient

   (A) au moins un polymère fixant les cheveux,
   (B) 10 à 40 % en poids d'au moins un alcool ayant de 1 à 4 atomes de carbone,
   (C) 10 à 30 % en poids d'eau,
   (D), 1 à 30 % en poids d'au moins un co-solvant volatil choisi parmi l'acétone, l'acétate de méthyle et un mélange de ceux-ci,
   (E) 20 à 75 % en poids d'un mélange de propulseurs pour aérosol à basé d'éther diméthylique et d'hydrocarbures fluorés.

2. Laque pour cheveux selon la revendication 1, **caractérisée en ce qu'**elle contient en tant que propulseurs pour aérosol 15 à 45 % en poids d'éther diméthylique et 5 à 30 % en poids d'au moins un hydrocarbure fluoré.

3. Laque pour cheveux en aérosol selon la revendication 2, dans laquelle la composition contient

   (A) au moins un polymère fixant les cheveux,
   (B) 20 à 30 % en poids d'au moins un alcool ayant de 1 à 4 atomes de carbone,
   (C) 15 à 25 % en poids d'eau,
   (D) 5 à 15 % en poids d'au moins un co-solvant volatil choisi parmi l'acétone, l'acétate de méthyle et un mélange de ceux-ci,
   (E) 25 à 35 % en poids d'éther diméthylique et 5 à 15 % en poids d'au moins un hydrocarbure fluoré.

4. Laque pour cheveux selon l'une quelconque des revendications précédentes, **caractérisée en ce que** le système de pulvérisation comporte une valve à orifice latéral.

5. Laque pour cheveux selon l'une quelconque des revendications précédentes, **caractérisée en ce que** le système de pulvérisation est configuré de sorte que le débit de sortie du produit va de 3,5 à 5,5 g pour 10 secondes de durée de pulvérisation.

6. Laque pour cheveux selon l'une quelconque des revendications précédentes, **caractérisée en ce que** le polymère (A) est contenu à raison de 3 à 20 % en poids.

7. Laque pour cheveux selon l'une quelconque des revendications précédentes, **caractérisée en ce que** l'alcool (B) est choisi parmi l'éthanol et l'isopropanol.

8. Laque pour cheveux selon l'une quelconque des revendications précédentes, **caractérisée en ce que** l'hydrocarbure fluoré est choisi parmi le 1,1-difluoroéthane et le 1,1,1,2-tétrafluoroéthane.

9. Laque pour cheveux selon l'une quelconque des revendications précédentes, **caractérisée en ce qu'**elle contient de l'acétone en tant que co-solvant (D).

10. Laque pour cheveux selon l'une quelconque des revendications précédentes, **caractérisée en ce qu'**elle ne contient pas plus de 55 % en poids de substances classées comme VOC (composés organiques volatils).

11. Utilisation d'acétone, d'acétate de méthyle ou d'un mélange de ceux-ci, pour l'amélioration des propriétés concernant l'utilisation de laques pour cheveux en aérosol, aqueuses-alcooliques, qui contiennent un mélange de propulseurs pour aérosol à base d'éther diméthylique et d'hydrocarbures fluorés.